Europäisches Patentamt

European Patent Office

Office européen des brevets

(11) Publication number: **0 374 947 B1**

# EUROPEAN PATENT SPECIFICATION

(45) Date of publication of patent specification: **08.03.95**

(51) Int. Cl.⁶: **C07C 331/28**, C07D 257/02, C07B 59/00, C07F 15/00, C07F 19/00, A61K 51/04

(21) Application number: **89123768.7**

(22) Date of filing: **22.12.89**

The file contains technical information submitted after the application was filed and not included in this specification

(54) **Process for preparing isothiocyanato functionalized metal complexes.**

(30) Priority: **23.12.88 US 289172**
**20.07.89 US 383103**

(43) Date of publication of application:
**27.06.90 Bulletin 90/26**

(45) Publication of the grant of the patent:
**08.03.95 Bulletin 95/10**

(84) Designated Contracting States:
**BE CH DE ES FR GB GR IT LI NL**

(56) References cited:
**EP-A- 0 292 689          EP-A- 0 296 522**
**EP-A- 0 353 450          WO-A-84/03698**
**WO-A-86/06384            US-A- 3 994 966**
**US-A- 4 622 420          US-A- 4 647 447**
**US-A- 4 994 560**

(73) Proprietor: **THE DOW CHEMICAL COMPANY**
**2030 Dow Center,**
**Abbott Road**
**Midland,**
**Michigan 48640 (US)**

(72) Inventor: **Fazio, Michael J.**
**4617 Forestview Drive**
**Midland**
**Michigan 48640 (US)**
Inventor: **Pollock, Douglas K.**
**4526 James Drive**
**Midland**
**Michigan 48640 (US)**
Inventor: **Kotite, Nicolas J.**
**2600 Abbott Road**
**Midland**
**Michigan 48640 (US)**

CHEMICAL ABSTRACTS, vol. 102, no. 6, February 11, 1985, Columbus, Ohio, USA; C. F. MEARES et al, "Conjugation of antibodies with bifunctional chelating agents: isothiocyanate and bromoacetamide reagents, methods of analysis, and subsequent addition of metal ions", page 322, column 2, abstract no. 50 880g

R. T. Morrison and R. N. Boyd, ORGANIC CHEMISTRY, 1969, Allyn and Bacon, Inc., Boston, USA; pages 751, 758-760, chapter 23

ORGANIC CHEMISTRY, 6th edition, 1962, Barnes & Noble, Inc., New York, N.Y., USA, page 144

INORGANIC CHEMISTRY, vol. 8, no. 4, April 1969, J. H. FORSBERG et al, "Observations on the rare earths. LXXIX. The syntheses and properties of ethylenediamine chelates of the tripositive lanthanide ions", pages 883-888

(74) Representative: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**Description**

The present invention concerns a novel process for preparing isothiocyanato fuctionalized metal complexes. The complexes formed are bifunctional compounds which are useful as various therapeutic and/or diagnostic agents.

Functionalized chelants, or bifunctional coordinators, are known to be capable of being covalently attached to an antibody having specificity for cancer or tumor cell epitopes or antigens. Radionuclides complexes of such antibody/chelant conjugates are useful in diagnostic and/or therapeutic applications as a means of conveying the radionuclide to a cancer or tumor cell. See, for example, Meares et al., *Anal. Biochem*. 142, 68-78 (1984); and Krejcarek et al., *Biochem. and Biophys. Res. Comm*. 77, 581-585 (1977).

The present invention concerns a process for preparing isothiocyanto fuctionalized metal complexes. A radionuclide can be used, and is prefered, in these complexes.

Isothiocyanto functionalized ligands are reported in the literature and are being used to conjugate radioactive isotopes to antibodies. For example see Gansow et al., *Inorg. Chem*. 25, 2772-81 (1986); Meares et al., *Analytical Biochem*. 142, 68-78 (1984); U. S. Patent 4,454,106.

The methodology taught in the art to prepare such complexes involves treatment of an antibody/chelant conjugate with the radionuclide to form a complex followed by purification of the complex. A major disadvantage of such methodology is that the radionuclide (a lanthanide or transition metal) must be kinetically labile in order to be rapidly sequestered by the antibody/chelant conjugate.

Another disadvantage associated with the use of labile radionuclides for antibody labelling is that substitutionally labile trace metals (which are not radioactive) are frequently incorporated into the chelate. Competition for such non-active trace metals diminishes the biological efficacy of the antibody/chelate complex since a lower quantity of radionuclide is delivered to the target site.

EP-A 0 292 689 teaches the production of a 1,4,7-tris-(carboxymethyl)-10-(4-isothiocyanato)benzyl-1,4,7,10-tetraazacyclododecanato-functionalized metal complex, wherein the complexed metal ion is gadolinium and complexing is effected with tertiary amine groups only. EP-A 0 353 450 (publication date 02/07/90) discloses a method for activating aminofunctionalized samarium 153 complexes, by reacting the aminofunctionalized samarium 153 chelate with thiophosgene in the presence of water and chloroform. EP-A 0 296 522 (publication date 12/28/88) discloses a process, wherein aminofunctionalized chelates are reacted with thiophosgene to prepare the isothiocyanate. For example, $^{105}$Rh(6-[(4-isothiocyanato phenyl)-methyl]-1,4,8,11-tetraazaundecane is produced by mixing thiophosgene and $^{105}$Rh(6-[(4-aminophenyl)-methyl]-1,4,8,11-tetraazaundecane.

Mikoler et al., European published application 139,675, teach the preparation of isothiocyanate fuctionalized chelates which can subsequently be conjugated to bio-organic molecules, e.g. haptens, antigens and antibodies. These complexes are prepared by chelating the isothiocyanate functionalized ligand.

In contrast, the present invention is directed to a novel process for preparing isothiocyanate compounds which comprises reacting thiophosgene with an amino functionalized polyaza chelate containing only primary and secondary amines complexed with rhodium, wherein a mixture of polar solvents or a single polar solvent is present. The present process concerns the preparation of the isothiocyanato function on a ligand after the metal has been chelated with the ligand. The formation of the isothiocyanto moiety on the ligand after the complex has been formed is important for several reasons: (1) when the complex requires heating or severe extremes in pH to form the complex, such as with rhodium or lanthanide macrocycles, the present process avoids the destruction of the isothiocyanate fuctionality during chelation; (2) when there are other primary or secondary amines present in the ligand, formation of the isothiocyanate fuctionalized ligand prior to chelation is impractical due to side reactions; (3) by forming the complex at as early a stage in the reaction as possible, complexation of undesired metals is reduced, and thus purity of the final product is enhanced; and (4) by forming the complex prior to the introduction of the isothiocyanate, purification of the complex, such as by ion exchange chromatography, is simplified and the added complication of the hydrolysis of the isothiocyanate during purification is also reduced.

Suprisingly, the present process provides a process to prepare isothiocyanates by thiophosgenation of amino fuctionalized chelates which results in a process that is rapid with high yield and provides a product having low metal contamination present. As there are a fewer number of reactions required overall by the present process on the ligand prior to chelation, the amount of undesired metal contamination is reduced.

The ligands which are used in the process of this invention are polyaza chelants. Examples of some of these polyaza chelants are given in Table III and are named as follows:

III A is 3-[(4-aminophenyl)methyl]-1,5,8,12-tetraazacyclotetradecane, the preparation of which is given in European published Application 296,522, published December 28, 1988;

III B is 6-[(4-aminophenyl)methyl]-1,4,8,11-tetraazaundecane, the preparation of which is given in European published Application 296,522, published December 28, 1988;

III D is 6-(3-aminopropyl)-1,4,7,11-tetraazaundecane, the preparation of which is given in European published Application 296,522, published December 28, 1988.

TABLE III

III A

III B

III D

The thiophosgene is added in excess to the mixture. The amount of excess used depends on the concentration of the starting amino fuctionalized chelate. The lower the concentration of chelate, the larger the excess of thiophosgene, to insure the rapid and complete conversion of amine to the isothiocyanate. For example, if the concentration of chelate is $10^{-3}$M, the ratio of thiophosgene to chelate is 5-20:1; if the concentration of chelate is $10^{-8}$M, the ratio of thiophosgene to chelate is several thousand times higher (i.e. $10^5$:1). The excess thiophosgene is removed by conventional techniques such as evaporation, chromatrography or extraction.

The process is run in a mixture of solvents such as water and a non-reactive solvent such as, for example, water/acetonitrile, water/dimethylformamide, water/chloroform, water/tetrohydrofuran, water/methylene chloride, water/ethanol, water/dioxane and water/butanol. The solvent can be a single phase or two phase system, but it is desirable that the complex be in solution.

The pH of the reaction may be from 2 to 10, preferrably from 6 to 8. The pH stability of the complex may restrict the operable pH range. Some complexes, such as ethylenediaminetetraacetic acid chelates of

4

lanthanides, are not very stable at pH 2. Additional base can be used to maintain the pH in the desired range or conventional buffers can be used.

The time of reaction when carried out with excess thiophosgene is very fast and usually complete after 5 to 10 minutes at room temperature (15 to 25°C). Higher or lower temperatures can be used (e.g. 0 to 50°C) but room temperature is preferred. Ambient pressure is used although higher or lower pressures can be employed. Pressure is not a critical feature of the present process.

The yield for the process is at least 50percent by weight.

Although any metal, whether a radioactive metal or not, can be used which complexes with the amino fuctionalized chelant. The complexes formed should have reasonable stability such that the metal complex is not readily dissociated. Complexes with stability constants of $10^5$ should be suitable. The radionuclides are prefered because of the use of the resulting products in a pharmaceutical drug for therapy and/or diagnosis. Especially preferred radioactive isotopes are those of samarium (Sm-153), holmium (Ho-166), ytterbium (Yb-175), lutetium (Lu-177), gadolinium (Gd-159), yttrium (Y-90), rhodium (Rh-105), indium (In-111), and technecium (Tc-99m).

Preparation of Starting Materials

Some of the chemicals used were obtained commercially from various sources, such as thiophosgene was from Aldrich Chemicals.

The preparation of many of the starting materials for this process can be found in the literature.

Radionuclides can be produced in several ways. In a nuclear reactor a nuclide is bombarded with neutrons to obtain a radionuclide.

Another method of obtaining radionuclides is to bombard nuclides with particles produced by a linear accelerator or a cyclotron. Yet another way is to isolate the radionuclide from a mixture of fission products. The method of obtaining the nuclides employed in the present invention is not critical thereto.

The present process has been used to make valuable synthetic precursors for radioactive pharmaceuticals. [See European Patent Application 89 111 497.7 and European published Application 296,522, published December 28, 1988.]

In the following examples, the following terms and conditions were used unless otherwise specified.

Glossary

BA-2,3,2-tet means 6-[(4-aminophenyl)methyl]-1,4,8,11-tetraazaundecane;
BITC-2,3,2-tet means 6-[(4-isothiocyanatophenyl)methyl]-1,4,8,11-tetraazaundecane;
HEPES means N-2-hydroxyethylpiperazine-N'-2-ethane sulfonic acid; and
TLC means thin layer chromotography.

General Experimental

Mass spectra were obtained on a VG ZAB-MS high resolution mass spectrometer (fast atom bombardment with xenon, using 3:1 dithiothreitol:dithioerythritol).

$R_f$ values are reported using these solvent systems and commercially available, normal phase, silica TLC plates (GHLF 250 micron, Analtek™ Inc.).

The following HPLC system was used for analyses and sample separations:

System I consisted of LKB 2150 pump, and 2152 controller, a UV detector - LKB 2238 UV Cord, a Berthold™ LB 506 A HPLC Radioactivity Monitor (of the International Berthold Group) and a Gilson™ Fraction collector 201-202 (Gilson™ International, Middleton, WI).

All percentages are by weight unless stated otherwise.

The invention will be further clarified by consideration of the following examples, which are intended to be purely exemplary of the use of this invention.

EP 0 374 947 B1

Process of the Invention

Example 1

Preparation of [Rh(BITC-2,3,2-tet)Cl$_2$]$^+$.

[Rh(BA-2,3,2-tet)Cl$_2$]$^+$ (10 mg) was dissolved in a mixture of 5 ml of pH 7 phosphate buffer (0.3M), 0.5 ml of acetonitrile, and 1 g of sodium chloride. The reaction mixture was stirred at room temperature (about 22°C) and 10μl of thiophosgene was added. After 15 minutes the hazy mixture was centrifuged, the yellow solid was washed with acetonitrile and centrifuged. The acetonitrile solution was stripped at reduced pressure to yield 3.1 mg of [Rh(BITC-2,3,2-tet)Cl$_2$]$^+$; The aqueous phase of the mixture, after centrifugation, was loaded on to a Chrom-Prep column washed with saturated sodium chloride, then water and eluted with acetonitrile. The acetonitrile fraction was then concentrated at reduced pressure to yield 5.6 mg of the desired product, overall yield is 80 percent.

Example 2

Preparation of [$^{105}$Rh(BITC-2,3,2-tet)Cl$_2$]$^+$.

Ten μl of freshly made thiophosgene solution (10 μl of thiophosgene in 5 ml of 90percent acetonitrile) was added to 400 μl of a solution of [$^{105}$Rh(BA-2,3,2-tet)Cl$_2$]$^+$ in 90 percent acetonitrile. The solution was mixed immediately and then allowed to stand at room temperature (about 22°C) for 20 minutes. The reaction mixture was then placed in a heating block (about 37°C). Excess unreacted thiophosgene as well as the solvent were evaporated by a gentle jet of N$_2$ for one hour. The dry [$^{105}$Rh(BITC-2,3,2-tet)Cl$_2$]$^+$, yield >95percent, is free from any unreacted thiophosgene.

Other embodiments of the invention will be apparent to those skilled in the art from a consideration of this specification or practice of the invention disclosed herein. It is intended that the specification and examples be considered as exemplary only.

**Claims**

1. A process for preparing isothiocyanate compounds which comprises reacting thiophosgene with an amino functionalized polyaza chelate selected from the group consisting of
    3-[(4-aminophenyl)methyl)-1,5,8,12-tetraazacyclotetradecane;
    6-[(4-aminopnenyl)methyl]-1,4,8,11-tetraazaundecane; or
    6-(3-aminopropyl)-1,4,7,11-tetraazaundecane.
   comlexed with rhodium, wherein a mixture of polar solvents is present.

2. The process of Claim 1 wherein the mixture of solvents is water with ethanol, butanol, acetonitrile, dimethylformamide, methylene chloride, tetrahydrofuran, chloroform or dioxane.

3. The process of Claim 1 wherein the pH of the reaction is from 2 to 10.

4. The process of Claim 1 wherein the temperature is from 0 to 50°C.

5. The process of Claim 1 for preparing [$^{105}$Rh(BITC-2,3,2-tet)Cl$_2$]$^+$ which comprises reacting [$^{105}$Rh(BA-2,3,2-tet)Cl$_2$]$^+$ with thiophosgene in water/acetonitrile at room temperature.

6. The process of Claim 1 for preparing [$^{105}$Rh(BITC-2,3,2-tet)Cl$_2$]$^+$ which comprises reacting [$^{105}$Rh(BA-2,3,2-tet)Cl$_2$]$^+$ with thiophosgene in water/acetonitrile at room temperature and removing the solvents by evaporation and nitrogen stream.

**Patentansprüche**

1. Verfahren zum Herstellen von Isothiocyanat-Verbindungen, umfassend das Umsetzen von Thiophosgen mit einem aminofunktionalisierten Polyazachelat, ausgewählt aus der, aus 3-[(4-Aminophenyl)methyl]-1,5,8,12-tetraazacyclotetradecan, 6-[(4-Aminophenyl)methyl]-1,4,8,11-tetraazaundecan oder 6-(3-Aminopropyl)-1,4,7,11-tetraazaundecan bestehenden Gruppe, welches mit Rhodium chelatisiert ist, worin ein

6

Gemisch von polaren Lösungsmitteln vorliegt.

2. Verfahren nach Anspruch 1, worin das Gemisch von Lösungsmitteln Wasser mit Ethanol, Butanol, Acetonitril, Dimethylformamid, Methylenchlorid, Tetrahydrofuran, Chloroform oder Dioxan ist.

3. Verfahren nach Anspruch 1, worin der pH der Reaktion 2 bis 10 ist.

4. Verfahren nach Anspruch 1, worin die Temperatur 0 bis 50 °C ist.

5. Verfahren nach Anspruch 1 zum Herstellen von $[^{105}Rh(BITC-2,3,2-tet)Cl_2]^+$, umfassend das Umsetzen von $[^{105}Rh(BA-2,3,2-tet)Cl_2]^+$ mit Thiophosgen in Wasser/Acetonitril bei Raumtemperatur.

6. Verfahren nach Anspruch 1 zum Herstellen von $[^{105}Rh(BITC-2,3,2-tet)Cl_2]^+$, umfassend das Umsetzen von $[^{105}Rh(BA-2,3,2-tet)Cl_2]^+$ mit Thiophosgen in Wasser/Acetonitril bei Raumtemperatur und Entfernen des Lösungsmittels durch Verdampfen und Stickstoffstrom.

**Revendications**

1. Procédé de préparation de composés de type isothiocyanate comprenant la réaction du thiophosgène avec un agent chélatant polyazoté à fonction amino choisi dans le groupe constitué de
3-[(4-aminophényl)méthyl]1,5,8,12-tétra-azacyclotétradécane,
6-[(4-aminophényl)méthyl]-1,4,8,11-tétraazaundécane, ou
6-(3-aminopropyl)-1,4,7,11-tétraazaundécane,
sous forme de complexe avec le rhodium, en présence d'un mélange de solvants polaires.

2. Procédé conforme à la revendication 1 dans lequel le mélange de solvants est un mélange d'eau avec de l'éthanol, du butanol, de l'acétonitrile, du diméthylformamide, du chlorure de méthylène, du tétrahydrofurane, du chloroforme ou du dioxane.

3. Procédé conforme à la revendication 1 dans lequel le pH de la réaction est compris entre 2 et 10.

4. Procédé conforme à la revendication 1 dans lequel la température est comprise entre 0 et 50 °C.

5. Procédé conforme à la revendication 1 pour la préparation de $[^{105}Rh(BITC-2,3,2-tet)Cl_2]^+$ consistant à faire réagir du $[^{105}Rh(BA-2,3,2-tet)Cl_2]^+$ avec du thiophosgène dans un mélange eau/acétonitrile à température ambiante.

6. Procédé conforme à la revendication 1 pour la préparation de $[^{105}Rh(BITC-2,3,2-tet)Cl_2^+$ consistant à faire réagir du $[^{105}Rh(BA-2,3,2-tet)Cl_2]^+$ avec du thiophosgène dans un mélange eau/acétonitrile à température ambiante et à éliminer les solvants par évaporation et par un flux d'azote.

7